# EUROPEAN PATENT SPECIFICATION

(11) **EP 3 717 051 B1**
(45) Date of publication and mention of the grant of the patent: **29.10.2025**
(21) Application number: 18933250.5
(22) Date of filing: 14.09.2018
(51) Int. Cl.: A61M 25/01, A61B 1/01

(54) **INTRAVASCULAR IMAGING SYSTEM WITH FORCE ERROR DETECTION AND REMEDIATION**
INTRAVASKULÄRES BILDGEBUNGSSYSTEM MIT KRAFTFEHLERERKENNUNG UND -BEHEBUNG
SYSTÈME D'IMAGERIE INTRAVASCULAIRE AVEC DÉTECTION ET CORRECTION D'ERREUR DE FORCE

(43) Date of publication of application: 07.10.2020
(73) Proprietor: Infraredx, Inc., Burlington, MA 01803 (US)
(72) Inventor: BARONE, David, Lexington, MA 02421 (US); BITNER, Todd, Waltham, MA 02452 (US); BECK, John, N., Hopkinton, MA 01748 (US)
(74) Representative: Karl, Christof
(86) International application number: PCT/US2018/051121
(87) International publication number: WO 2020/055427

(56) References cited:
- WO-A2-2009/092059
- US-A1- 2005 049 574
- US-A1- 2012 071 752
- US-A1- 2012 071 752
- US-A1- 2015 297 864

## Description

### FIELD OF THE INVENTION

The present invention relates generally to catheters with movable cores and, more particularly, to systems for detecting and remediating force error conditions in such catheters.

### BACKGROUND

Catheters with imaging components are commonly used to inspect vessels in the body. Such catheters may include a hollow, tubular sheath made of a flexible material. The sheath may be placed in a vessel using a guidewire and used as a conduit for positioning imaging components in the vessel. For example, imaging components may be mounted at the distal end of a torque cable and advanced into the vessel via the sheath. Depending on the type of information desired, the imaging components may include optical elements such as mirrors or lenses for transmitting and receiving light energy (e.g., near infrared light) and/or transducers for transmitting and receiving sound energy (e.g., ultrasonic pulses). Imaging components may be mounted on an imaging tip at the distal end of a torque cable, and the torque cable may be moved relative to the sheath to obtain information about the vessel. In one example, the imaging tip may be advanced distally to a desired position in the sheath (e.g., a start position) and may then be retracted proximally and rotated to obtain a 360 degree view along a desired portion of the vessel.

The imaging tip at the distal end of the torque cable must be long enough to accommodate one or more imaging sensors and is typically much more rigid than the outer sheath. Thus, if the imaging tip is advanced distally into a portion of the outer sheath that is kinked or tightly curved, there is a possibility of the imaging tip not being able to traverse the outer sheath. In extreme cases, there is also a possibility of the imaging tip damaging the outer sheath or even penetrating though the outer sheath and creating a risk of injury.

WO 2009/092059 A2 discloses a system for remotely controlling the positioning within the body of a patient of an elongated medical device optionally having a control handle, comprises a robotic system and a remote controller configured to control the robotic device. The robotic system comprises a handle controller; a sled member coupled to the handle controller, the sled member being configured to position the medical device within the body of the patient; and a sled base configured to advance the sled member towards the body of a patient, the sled bed being coupled to a sterile barrier effective to maintain sterility inside the sled base. A medical device introducer is effective to guide the elongated medical device into a patient's body.

### SUMMARY OF THE INVENTION

The invention is defined by the appended claims. The dependent claims define advantageous embodiments. According to the invention, a pullback and rotation unit with force error detection and remediation is provided for axially translating and rotating a torque cable in an elongate outer sheath of a catheter. The pullback and rotation unit includes a housing having a longitudinal axis, and a rotor that is disposed in the housing and mounted to be rotatable and linearly translatable relative to the longitudinal axis of the housing. The rotor is further configured to be coupled with a proximal end of the torque cable. The pullback and rotation unit also includes at least one sensor configured to provide an output indicative of an axial force exerted by the torque cable on the rotor in the pullback and rotation unit. The pullback and rotation unit also includes a controller configured to, when the output exceeds a predetermined threshold, cause the rotor to automatically perform a remedial action to relieve at least a portion of the axial force, wherein the controller is configured to cause the rotor, when performing the remedial action, to retract proximally a predetermined distance. The pullback and rotation unit may alleviate problems associated with kinking of an outer sheath in an imaging catheter system and other force error conditions preventing or inhibiting distal translation of the torque cable in the outer sheath.

In an embodiment, the at least one sensor may be mounted on the rotor in the pullback and rotation unit. For example, the at least one sensor may be mounted between a coupling on the rotor configured to couple with the proximal end of the torque cable and a main body of the rotor. These embodiments may improve sensitivity by reducing intervening components between the sensor(s) and the torque cable.

In an embodiment, the coupling may be mounted to move longitudinally relative to the main body of the rotor. This embodiment may facilitate transfer of forces from the coupling to the sensor(s) as opposed to the main body of the rotor.

In an embodiment, the sensor(s) may be mounted on the main body of the rotor. This embodiment may reduce manufacturing costs by allowing the sensor(s) to be positioned near electrical connections in the main body of the rotor.

In an embodiment, the system includes a plurality of sensors, which may improve reliability. The plurality of sensors may be radially spaced from an axis of rotation of the rotor, which may improve the ability of the sensors to detect forces applied to the coupling, particularly when the coupling is mounted to float longitudinally relative to the main body of the rotor.

In an embodiment, the output is an average of the outputs from one or more sensors over a period of time, which can improve reliability of the system and help reduce false alarms. The period of time may be at least one full rotation to further improve reliability.

In an embodiment, the predetermined threshold may correspond to a force greater than an average axial force applied to the coupling during normal operation of the catheter. This embodiment may improve reliability and reduce the occurrence of false alarms. The predetermined threshold may also be set below a level at which damage to the catheter components, such as the outer sheath or the imaging tip, may occur, thereby increasing the possibility that the procedure may be resumed after the error condition is addressed.

According to the invention, the controller is configured to cause the rotor, when performing the remedial action, to retract proximally a predetermined distance. The invention thus may relieve at least a portion of the axial force applied to the rotor by the torque cable, reducing the possibility of damaging the system and possibly allowing the procedure to be resumed. The controller may be configured to cause the rotor, in the remedial mode, to continue rotating as it is retracted, which can prevent binding of the torque cable in the outer sheath under certain circumstances.

In a second aspect of the invention, an intravascular imaging catheter system includes a pullback and rotation unit according to the first aspect of the invention, and an imaging catheter including an elongate outer sheath having a proximal end coupled with the housing, a torque cable disposed in the outer sheath and having a proximal end coupled with the rotor, and an imaging tip located at a distal end of the torque cable. This aspect may alleviate problems associated with kinking of an outer sheath in an intravascular imaging catheter system and other error conditions preventing or inhibiting distal translation of the torque cable in the outer sheath.

In a non-claimed example, a method of imaging is provided using a catheter including an elongate outer sheath, a torque cable disposed in the outer sheath, and an imaging tip located at a distal end of the torque cable, wherein the imaging tip includes at least one imaging component selected from the group comprising an optical element and an ultrasonic transducer. The method includes the steps of coupling a proximal end of the elongate outer sheath with a housing of a pullback and rotation unit, coupling a proximal end of the torque cable with a rotor disposed within the housing of the pullback and rotation unit, inserting the catheter, linearly translating the imaging tip distally relative to the outer sheath by linearly translating the rotor distally in the housing of the pullback and rotation unit, receiving an output from at least one sensor indicative of an axial force applied to the rotor by the torque cable, and if the output from the at least one sensor exceeds a predetermined threshold, causing the rotor to automatically perform a remedial action to relieve at least a portion of the axial force. The method may alleviate problems associated with kinking of an outer sheath in an imaging catheter system and other error conditions preventing or inhibiting distal translation of the torque cable in the outer sheath.

In an embodiment, the at least one sensor may be mounted on a rotor in the pullback and rotation unit. This embodiment may reduce cost by implementing force detection and remediation in a portion of the system that is normally re-used. This arrangement may also facilitate compatibility with existing catheters.

In an embodiment, the at least one sensor may be mounted between a coupling on the rotor configured to couple with the proximal end of the torque cable and a main body of the rotor. This embodiment may improve sensitivity by reducing intervening components between the sensor(s) and the torque cable.

In an embodiment, the coupling may be mounted to move longitudinally relative to the main body of the rotor. This embodiment may facilitate transfer of forces from the coupling to the sensor(s) as opposed to the main body of the rotor.

In an embodiment, the sensor(s) may be mounted on the main body of the rotor. This embodiment may reduce manufacturing costs by allowing the sensor(s) to be positioned near electrical connections.

In an embodiment, the system includes a plurality of sensors, which may improve reliability. The plurality of sensors may be radially spaced from an axis of rotation of the rotor, which may improve the ability of the sensors to detect forces applied to the coupling, particularly when the coupling is mounted to float longitudinally relative to the main body of the rotor.

In an embodiment, the output is an average of the outputs from one or more sensors over a period of time, which can improve reliability of the system and help reduce false alarms. The period of time may be at least one full rotation to further improve reliability.

In an embodiment, the predetermined threshold may correspond to a force greater than an average axial force applied to the coupling during normal operation of the catheter. This embodiment may improve reliability and reduce the occurrence of false alarms. The predetermined threshold may also be set below a level at which damage to the catheter components, such as the outer sheath or the imaging tip, may occur, thereby increasing the possibility that the procedure may be resumed after the error condition is addressed.

According to a non-claimed example, the method causes the rotor, when performing the remedial action, to retract proximally a predetermined distance. This embodiment may relieve at least a portion of the axial force applied to the rotor by the torque cable, reducing the possibility of damaging the system and possibly allowing the procedure to be resumed. The method may also cause the rotor, in the remedial mode, to continue rotating as it is retracted, which can prevent binding of the torque cable in the outer sheath under certain circumstances.

### BRIEF DESCRIPTION OF THE DRAWINGS

Embodiments of the invention will be described with reference to the following figures, wherein identical structures, elements or parts which appear in more than one figure are labeled with the same reference number, and in which:
FIG. 1 is a perspective view of an imaging catheter for an imaging catheter system according to an example embodiment of the present invention.
FIG. 2 is an exploded view of an imaging catheter system including an imaging catheter, a pullback and rotation unit, and a console according to an example embodiment of the present invention.
FIG. 3 is an enlarged view of the imaging tip at the distal end of the imaging catheter shown in FIG. 2.
FIG. 4 is a side view of a rotor carriage assembly for pullback and rotation of an imaging tip in an imaging system according to an example embodiment of the present invention.
FIG. 5 is a sectional view of the rotor carriage assembly of FIG. 4, taken though line 5-5.
FIG. 6 is a side view of the rotor carriage assembly of FIG. 4 with an imaging catheter attached.
FIG. 7 is a sectional view of the rotor carriage assembly of FIG. 6, taken though line 7-7.
FIG. 8 is a partial sectional view of the rotor carriage assembly of FIG. 7, taken though line 8-8.
FIG. 9 is a flowchart illustrating a method of operating an imaging catheter system according to a non-claimed example.

### DETAILED DESCRIPTION

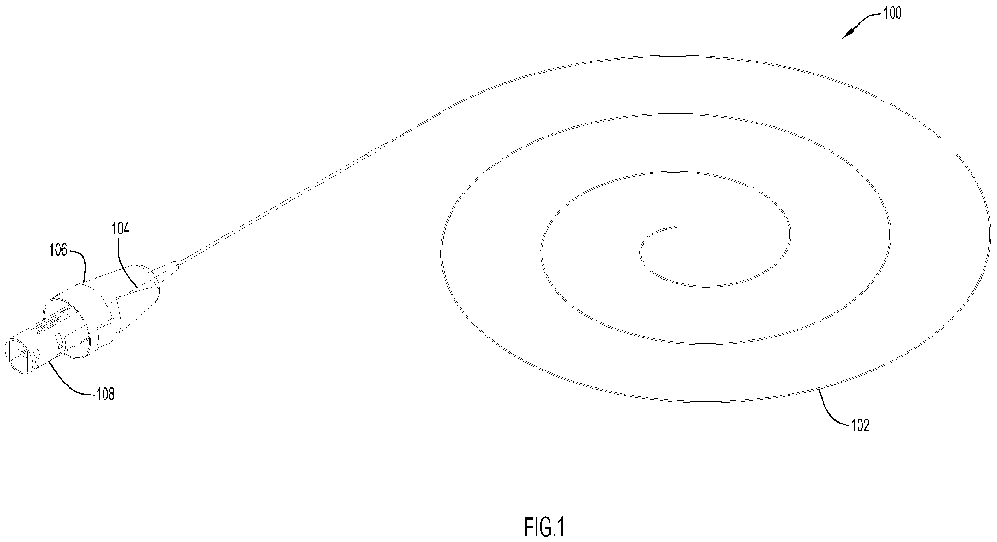

FIG. 1 is a perspective view of an imaging catheter **100** for an imaging catheter system with force error detection and remediation according to an example embodiment. Imaging catheter **100** includes an elongate tubular outer member or sheath **102** configured for insertion into the lumen of a vessel (e.g., using a guidewire), an elongate inner core in the form of a torque cable **104** configured to extend longitudinally within the outer sheath, and outer and inner hubs **106** and **108** at proximal ends of the outer sheath **102** and the torque cable **104,** respectively. In the example shown in FIG. 1, the outer hub **106** is hollow, and the inner hub **108** is disposed at least partly within the outer hub and configured to be rotatable and linearly translatable relative to a longitudinal axis of the outer hub. The torque cable **104** extends distally from the inner hub **108** into the outer sheath **102.**

In an example embodiment, the outer sheath **102** is composed of a flexible material that is efficiently transmissive (i.e., transparent or translucent) to near infrared light to facilitate use of the imaging catheter for near infrared spectroscopy (NIRS). Such material may be, for example, petrothene or any suitable polymer that efficiently transmits near infrared light. Inside the outer sheath **102** there may be a transmission medium, such as saline or other fluid, to facilitate use of the imaging catheter for intravascular ultrasound (IVUS) imaging. If the transmission medium is also transparent to near infrared light, the imaging catheter may be used for both IVUS and NIRS imaging. Because the outer sheath **102** is composed of a flexible material, it may conform to the shape of the vessel into which it is inserted.

In an example embodiment, the torque cable **104** is constructed using one or more hollow coils of wire composed of a material, such as steel or titanium, capable of transmitting axial forces and torque along the length of the torque cable. Thus, the torque cable **104** may be linearly translated and rotated relative to the longitudinal axis of the outer sheath **102** via linear translation and rotation of the inner hub **108.** Due to the coiled structure, the torque cable **104** may also bend somewhat to conform to the shape of the outer sheath **102** inside a vessel. However, if the outer sheath **102** is kinked or positioned in a highly tortuous section of a vessel with tight bends, the torque cable **104** may not be capable of traversing the outer sheath, and a force may be applied by the torque cable against the inner wall of the outer sheath as the torque cable is advanced distally into such a kink or bend. The force applied by the torque cable **104** against the outer sheath **102** may cause damage to the outer sheath or components mounted on the torque cable. At the same time, an axial compression of the torque cable **104** may occur. While a torque cable constructed of one or more hollow coils of wire has been described, it will be appreciated that other types of torque cables can be used, including, but not limited to, thin-walled tubes, spiral-cut tubes, tubes with cutouts to increase flexibility, and other elongate tubular members capable of transmitting torque and being linearly translated through an outer sheath.

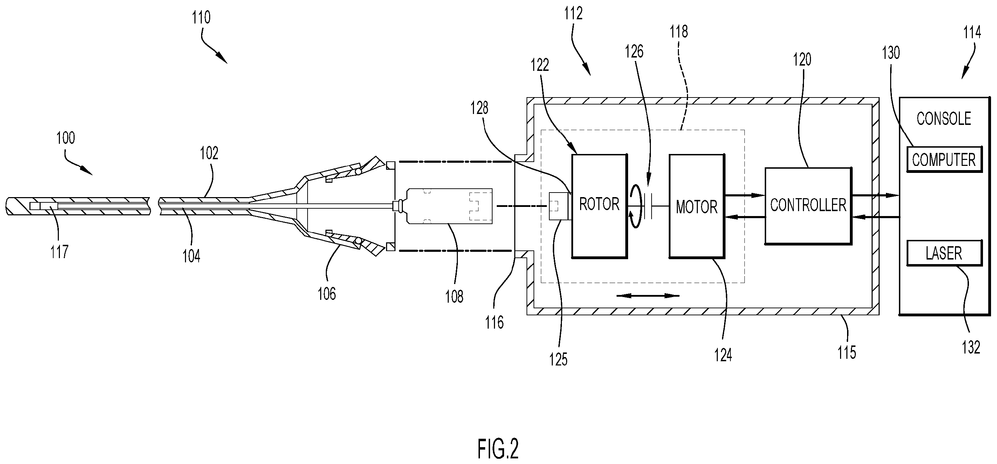

FIG. 2 is an exploded view of an example imaging catheter system **110** capable of detecting kinking and other problems with the catheter **100** based on axial compression of the torque cable **104** and taking remedial actions to prevent damage to the outer sheath **102** or other components of the catheter or the system. The imaging catheter system **110** includes an imaging catheter **100** as described above, a pullback and rotation unit (PBR) **112,** and a console **114.** The PBR **112** includes a housing **115** with a distal opening **116,** a rotor carriage **118** movable linearly along a longitudinal axis of the housing, and a controller **120** for controlling operation of the rotor carriage and communicating with the console **114.** Longitudinal travel of the rotor carriage **118** may be effected in a conventional manner, e.g., by means of a lead screw and rotary motor that powers the lead screw via a pulley and belt arrangement. The controller **120** may implemented in hardware (e.g., a circuit, an application specific integrated circuit, a field programmable gate array, etc.), software, or a combination thereof.

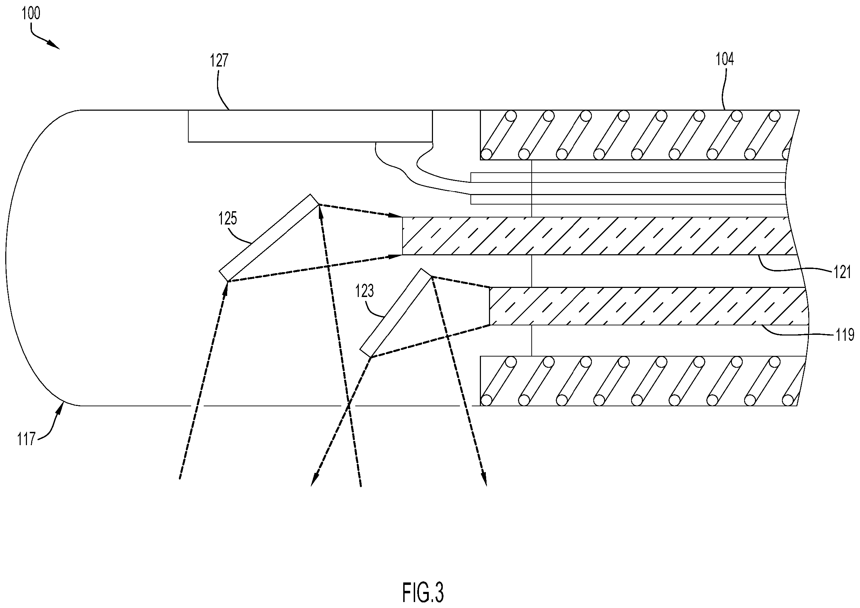

Referring now to FIGS. 2 and 3, it can be seen that the imaging catheter **100** includes an imaging tip **117** located at a distal end of the torque cable **104.** The imaging tip **117** includes at least one imaging component selected from the group comprising an optical element and an ultrasonic transducer. In the example shown, the imaging tip **117** includes optical elements to perform NIRS imaging and an ultrasonic transducer **127** to perform IVUS imaging. The optical elements include a delivery optical fiber **119** and a collection optical fiber **121** that extend between proximal and distal ends of the torque cable **104.** The distal ends of both the collection and delivery fibers are secured to the imaging tip **117** in alignment with other optical elements for guiding light to and from the optical fibers. To maintain proper alignment of the optical and/or ultrasonic components, the imaging tip **117** is preferably formed of a rigid material, such as a rigid plastic, metal, or ceramic material.

A near infrared light source such as a laser (see element 132 in FIG. 2) couples light into a proximal end of the delivery optical fiber **119** which guides the light distally to the reflective surface of a delivery mirror **123** located on imaging tip **117.** Mirror **123** is positioned to redirect the delivered light toward the vessel wall via the outer sheath **102.** A collection mirror **125,** also disposed on the imaging tip **117,** redirects light scattered from various depths of the vessel wall into the distal end of collection fiber **121** which transmits the collected light proximally in the catheter to an optical detector (not shown) for processing and analysis. Other light redirectors can be used in place of mirrors (e.g., prisms, bends in the optical fiber tips, etc.).

The optical detector that receives the collected light from collection fiber **121** produces an electrical signal that contains a spectral signature indicating the composition of the vessel wall and, in particular, whether the composition is consistent with the presence of lipids found in a vulnerable plaque. The spectral signature in the electrical signal can be analyzed using a spectrum analyzer (not shown) implemented in hardware, software, or a combination thereof.

The rotor carriage **118** includes a non-rotating portion **124** and a rotating portion or rotor **122** coupled with the non-rotating portion. Rotor **122** is configured to rotate about a longitudinal axis of the housing **115.** For example, a motor may be disposed on the non-rotating portion **124** and the rotor **122** may be mounted on a rotating drive shaft of the motor that is aligned with a longitudinal axis of the housing **115.** Rotor **122** includes a coupling **125** adjacent the distal opening **116** in the housing **115.** The coupling **125** is configured to mate with the inner hub **108** of the imaging catheter **100** to cause the inner hub (and thus the torque cable **104**) to rotate and translate linearly relative to the longitudinal axis of the outer sheath **102** in response to rotation and linear translation of the rotor **122** relative to the longitudinal axis of the housing **115.** Coupling **125** and inner hub **108** include mating connectors for communicating optical and/or electrical signals between the rotor **122** and imaging components on the torque cable **104.** Optical and/or electrical signals may be communicated between the rotor **122** and the non-rotating portion **124** via a slip ring **126** or other suitable rotary connector.

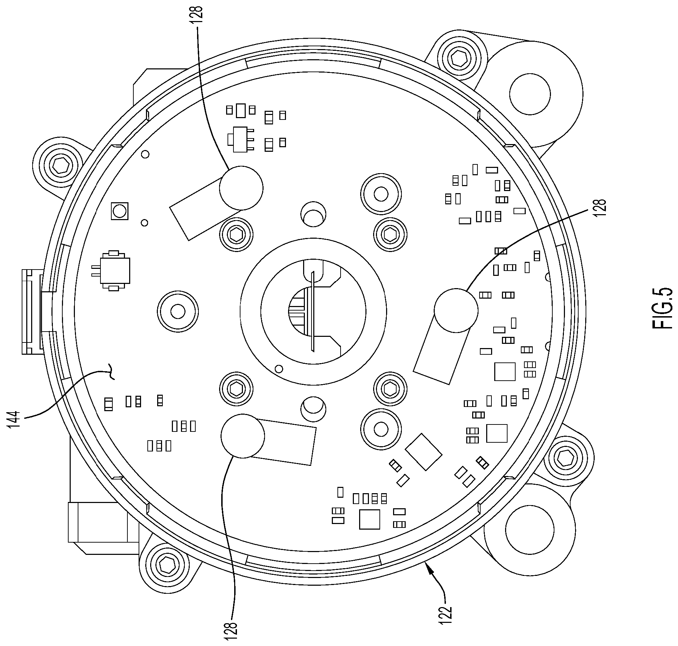

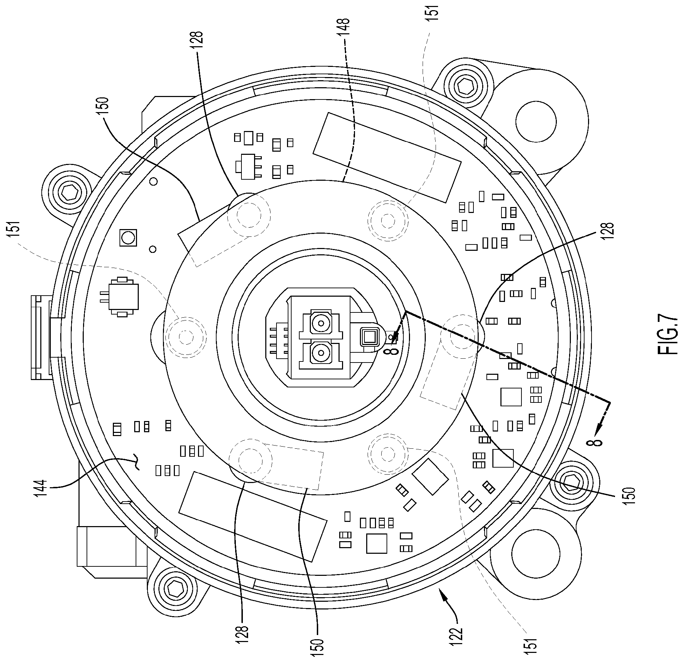

In an embodiment, one or more force or pressure sensors **128** may be located between the coupling **125** and another part of the rotor **122** to monitor compression of the torque cable **104,** which is seen as an axial force applied to the rotor in a proximal direction along the axis of rotation the rotor (or a longitudinal axis of the housing). For example, the rotor **122** may include a printed circuit board (PCB) with a planar surface oriented perpendicular to the axis of rotation of the rotor **122,** and the one or more sensors **128** may be mounted on a side of the PCB facing the coupling **125** (see, e.g., elements 128 in FIGS. 5 and 7). In an embodiment, the one or more sensors **128** may be superimposed with the coupling **125** (e.g., within a radius from the axis of rotation corresponding to a periphery of the coupling). The one or more sensors **128** may be disposed directly against the coupling **125,** or an intermediate member may be disposed between the one or more sensors and the coupling. In an alternative embodiment, the one or more sensors may be incorporated into the inner hub **108** (e.g., by forming the hub in two or more pieces and positioning the one or more sensors between axially spaced pieces of the hub). Output(s) from the one or more sensors **128** (e.g., voltage across a sensor) indicative of an axial force applied to the rotor **122** by the torque cable **104** may be communicated to the controller **120** via the slip ring **126** and the non-rotating portion **124** of the rotor. Examples of suitable force sensors include, but are not limited to, piezoelectric sensors, resistive sensors, capacitive sensors, electromagnetic sensors, and optical sensors.

Console **114** includes a computer **130** for communicating with the controller **120** in the PBR and a laser **132** for providing a source of near infrared light for NIRS imaging. It will be appreciated that the pullback and rotation unit **112** and the console **114** may be combined into a single unit. Alternatively, if the pullback and rotation unit **112** and console **114** are separate, some or all of the functions of the controller **120** may be performed by the console and vice versa.

In operation, catheter **100** is attached to PBR **112** by mating inner hub **108** with coupling **125** on the rotor **122** and mating outer hub 106 with the distal opening **116** on the housing **115.** After the system is powered on and initialized, the imaging tip **117** at the distal end of the torque cable **104** may be moved proximally and distally within the outer sheath **102** by moving the rotor **122** in the PBR **112** proximally and distally relative to the housing **115.** If, while moving distally, the imaging tip **117** at the distal end of the torque cable **104** encounters a kink or other obstruction in the outer sheath **102** that prevents or inhibits further distal movement, the torque cable will compress and start to bunch up, creating a spring force that is translated back to the coupling **125** on the rotor **122.** As a result, the coupling **125** is compressed against the force sensor(s) **128.** An output from the force sensor(s) (e.g., a voltage or current) changes as they are compressed, signaling to the controller **120** in the PBR that something is wrong. In an embodiment, the controller **120** may be configured such that, once an output indicative of a force threshold is reached, the controller causes linear motion of the rotor carriage **118** in the distal direction to stop and the rotor carriage to "recoil" (a quick move or retraction in the proximal direction) to relieve the spring force in the system due to compression of the torque cable. The controller **120** may also be configured to inform the user (e.g., via a display on the PBR and/or the computer in the console) that there may be a kink in the catheter or some other force error condition preventing or inhibiting distal movement and that they should retract the outer sheath slightly in order to ease re-advancement of the inner core. Once the force error condition is resolved, the procedure may be resumed. In an example embodiment, the catheter **110** may be removed from the PBR after the procedure, and the PBR may be re-used with a different catheter for a new procedure.

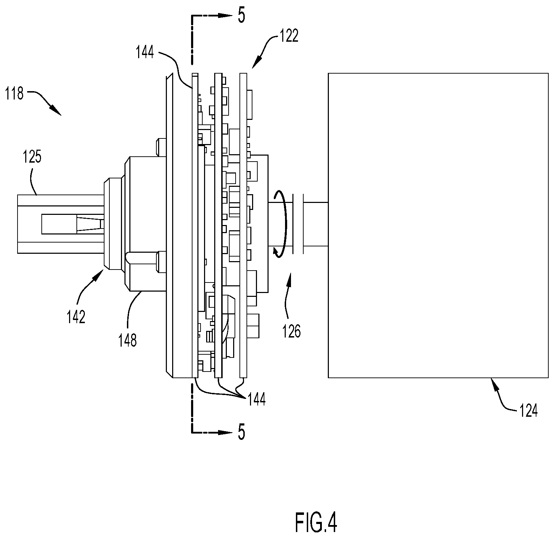

FIGS. 4 - 8 illustrate details of a rotor carriage **118** for pullback and rotation of an imaging tip according to an example embodiment. As noted above, the carriage **118** may be configured to be linearly translated in proximal and distal directions in a housing of a PBR. The rotor carriage **118** includes a non-rotating portion **124** and a rotating portion or rotor **122.** A slip ring **126** is disposed between the rotating and non-rotating portions of rotor carriage **118** to facilitate transmission of optical and/or electrical signals therebetween. Rotor **122** is configured to rotate about a longitudinal axis of rotation and includes a nosepiece **142** at a distal end and a main body or frame mounting one or more PCBs **144** at a proximal end. The PCBs **144** are oriented perpendicular to the axis of rotation of the rotor and are stacked against the nosepiece **142.** In the example embodiment shown, the PCB adjacent the nosepiece **142** is configured as a NIRS processing board, but it can be configured to have any desired function. As best seen in FIG. 5, three force sensors **128** are arranged on the distal side of the PCB facing the nosepiece **142.** The force sensors **128** are disposed in a circular arrangement and are equiangularly spaced from one another (e.g., at about 120 degree intervals). Utilizing a plurality of sensors increases reliability of the force error detection system, and arranging the sensors at equiangular locations along a path of rotation may further increase reliability.

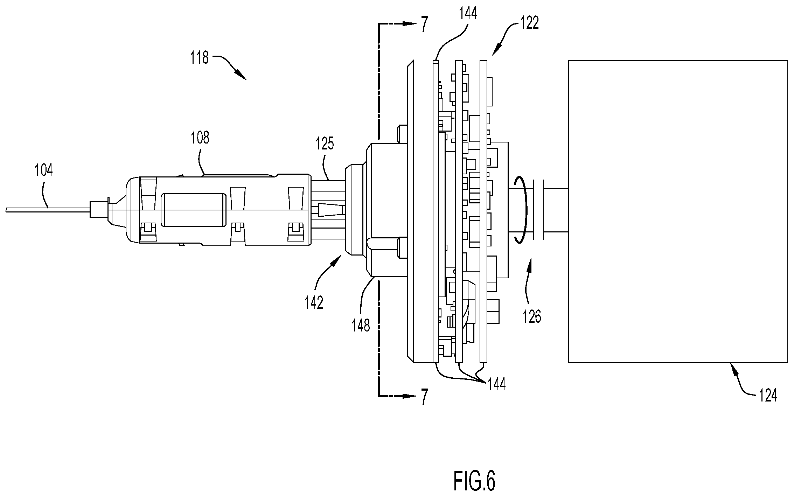

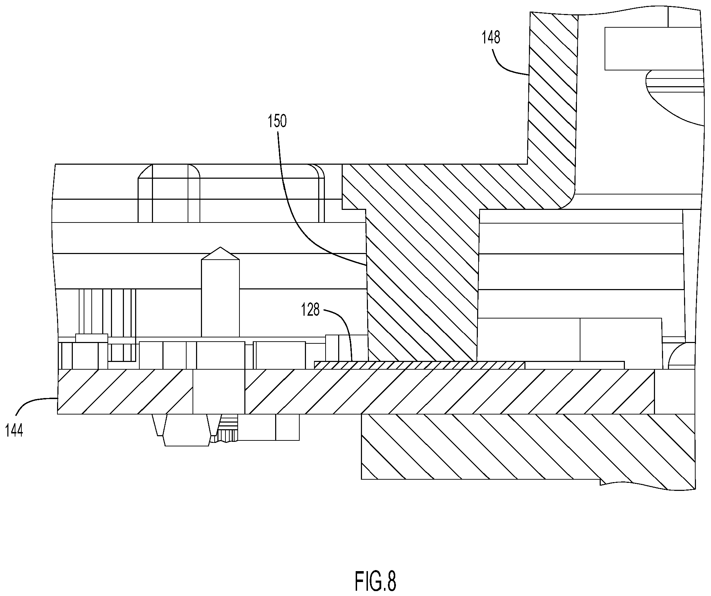

The nosepiece **142** includes a generally cylindrical collar **148** and a coupling **125** protruding distally from a center of the collar. As described above and shown in FIG. 6, the coupling **125** is configured to mate with an inner hub **108** at the proximal end of an imaging catheter. As best seen in FIG. 7, the sensors **128** are arranged to be superimposed with the collar **148** when viewed along the axis of rotation. That is, the sensors **128** are arranged along a circular path concentric with the collar **148** and having a radius about the same as or slightly smaller or larger than the collar. As best seen in FIG. 8, three legs **150** protrude proximally from the collar **148** towards the sensors **128.** The nosepiece **142,** including the coupling **125,** the collar **148,** and legs **150,** is preferably formed of a rigid material, such as a rigid metal or plastic material, but can be formed of any suitable material capable of transmitting forces between the torque cable and the force sensors. In an example embodiment, the nosepiece **142** is configured to "float" (that is, move distally a small amount) relative to the rest of the rotor **122.** For example, the nosepiece **142** may be mounted on the main body of the rotor **122** loosely (e.g., by installing fasteners **151** through the nosepiece into the main body of the rotor with a small axial gap (e.g., up to 0.5 mm) between a head of the fastener and the nosepiece). As a result, during operation, the legs **150** are free to move back and forth (i.e., proximally and distally) relative to the sensors **128** so that axial forces applied to the nosepiece by the torque cable are in turn applied to the sensors instead of the mounting. When an error condition occurs, such as when translating the torque cable **104** distally into a kinked portion of the outer sheath, the legs **150** are moved proximally against the force sensors **128.** Alternatively, the legs **150** can be formed of an elastic material and maintained in contact with the force sensors, or elastic members (such as springs or flexible spacers) can be mounted between the nosepiece and the board mounting the force sensors or other part of the rotor.

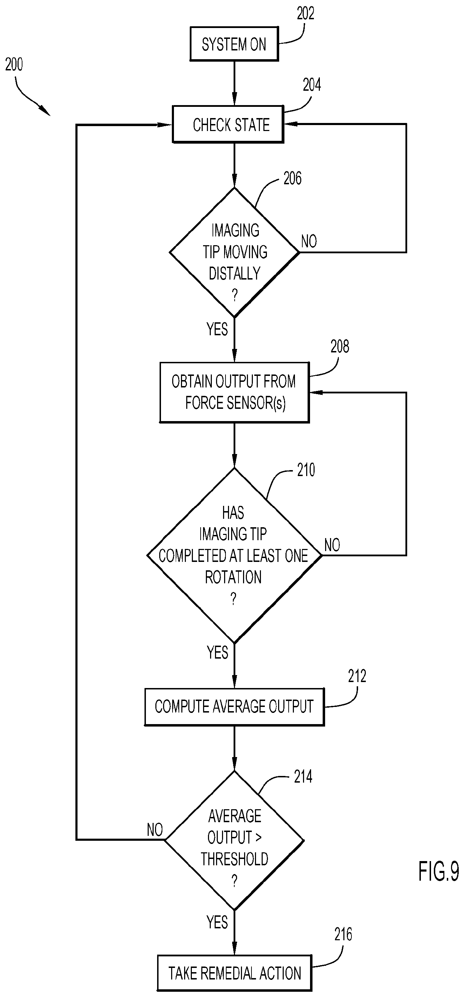

FIG. 9 is a flowchart illustrating a non-claimed method **200** of operating an imaging catheter system according to an example embodiment. The system is powered on at step **202.** The system (e.g., via the controller) checks the state of the imaging tip at step **204** and determines whether the imaging tip is moving distally at step **206** (e.g., by checking whether a linear actuator is moving the rotor carriage distally). If the imaging tip is not moving distally, the method returns to step **204.** If the imaging tip is moving distally, the system (e.g., via the controller) obtains output(s) from one or more force sensors positioned between the imaging tip and the PBR at step **208.** The outputs may be analog signals (e.g., voltage across a sensor) or digital signals or any other type of electrical signals.

The system (e.g., via the controller) determines whether the imaging tip has completed a predetermined number of rotations greater than or equal to one at step **210** (e.g., by monitoring a rotary encoder in the PBR housing, or by monitoring rotational speed of the rotor and time). If the imaging tip has not completed the predetermined number of rotations, the method returns to step **208.** If the imaging tip has completed the predetermined number of rotations, the system (e.g., via the controller) computes an average output at step **212.** For example, if a single sensor is used, the system may compute an average of all outputs obtained from the single sensor over the predetermined number of rotations. Or, if multiple sensors are used, the system may compute an individual average for each sensor over the predetermined number of rotations and a combined average using the individual averages. In an example embodiment, the average is a rolling average, although other types of averaging or filtering can be used.

The system (e.g., via the controller) compares the average output to a predetermined threshold at step **214.** In an example embodiment, the predetermined threshold may be set to correspond to a force indicative of a catheter malfunction. For example, the predetermined threshold may be set to correspond to a force indicative of the torque cable bunching up when the imaging tip encounters a kink or bend in the outer sheath that it cannot traverse. In such a case, the predetermined threshold may depend on the materials used for the catheter components and their configuration. Preferably, the predetermined threshold is set to correspond to a force below a level at which damage to the catheter components is likely to occur, so that remedial actions may be taken to relieve compression of the torque cable and continue use of the imaging catheter. In an example embodiment, the predetermined threshold may correspond to a force less than 10 N. In another example embodiment, the predetermined threshold may correspond to a force in the range of 2N - 6N.

If the average output is less than or equal to the predetermined threshold, the method returns to step **204.** If the average exceeds the predetermined threshold, the system takes remedial action at step **216.** For example, the system may automatically stop further linear translation of the imaging tip in the distal direction. The system also automatically retracts the imaging tip proximally a predetermined distance (e.g., 10 mm) to relieve compression of the torque cable. The system may stop rotation of the imaging tip or continue to rotate the imaging tip during retraction following a force error event. The system may also notify the user of the error condition (e.g., via a display on the PBR or via the computer in the console) so that the user may take remedial action (e.g., by retracting the outer sheath to eliminate the kink).

From the above, it will be appreciated that kinking of an outer sheath in an imaging catheter system and other force error conditions preventing or inhibiting distal translation of an imaging tip in the outer sheath can be addressed by mounting pressure or force sensors at various locations between the imaging tip and a PBR. A controller in the system may be configured to monitor an axial force exerted on the sensors in a proximal direction and to enter a force error state or mode when the imaging tip is moving distally in the sheath and the axial force measured by the sensors exceeds a predetermined threshold (e.g., suggesting that the linearly translating imaging tip is encountering a kink in the outer sheath or some other error condition preventing further linear translation). Since excessive force can indicate a dangerous condition, in the force error mode, the controller may be configured to cause one or more remedial actions to be performed.

While an example embodiment is shown in which three force sensors are spaced 120° apart on a NIR board in the PBR, it will be appreciated that other arrangements can be utilized. For example, fewer than three sensors or more than three force sensors may be utilized, although a plurality of sensors are preferred for greater reliability. If a plurality of sensors are used, they may be arranged in a uniform (e.g., equiangularly spaced) pattern or a non-uniform pattern. Also, the one or more force sensors may be positioned at various locations in the imaging catheter system, such as between the imaging tip and the torque cable, between the torque cable and the inner hub, between axially spaced portions of the inner hub, between the inner hub and the rotor coupling, and between parts of the rotor.

Furthermore, while an example embodiment is described in which the output used for force detection is an average output over a period of time, it will be appreciated that other algorithms may be used. For example, the output used for force detection may be an average output over a period of time as described above, or a sum of outputs over a period of time, or a maximum output over a period of time, or a median output over a period of time, or an output derived from fewer than all of the sensors (e.g., the highest two out of three, etc.), or an output that excludes outliers, or a combination of the foregoing.

In addition, while an imaging tip having optical and/or ultrasonic components is described, it will be appreciated that the imaging tip can include components for performing therapy, such as tissue ablation, in addition to or in lieu of imaging components.

As noted above, the predetermined threshold may correspond to a force indicative of a kink or other obstruction or condition of the catheter that prevents or inhibits distal movement of the tip relative to the sheath. For example, the predetermined threshold may correspond to a force greater than an average axial force applied to the coupling during normal operation of the catheter. In a preferred embodiment, the predetermined threshold is below a level at which damage to the catheter components or other parts of the system will occur.

The above-described embodiments are provided by way of example and are not intended to limit the scope of the invention. It should be understood that features described with respect to one embodiment may be used with other embodiments.

## Claims

1. A pullback and rotation unit (112) for axially translating and rotating a torque cable (104) in an elongate outer sheath (102) of a catheter (100), the pullback and rotation unit (112) comprising:
a housing (115) with a longitudinal axis;
a rotor (122) disposed in the housing (115) and mounted to rotate and linearly translate relative to the longitudinal axis of the housing (115), the rotor (122) further being configured to be coupled with a proximal end of the torque cable (104);
at least one sensor (128) configured to provide an output indicative of an axial force exerted by the torque cable (104) on the rotor (122); and
a controller (120) configured to, when the output exceeds a predetermined threshold, cause the rotor (122) to automatically perform a remedial action to relieve at least a portion of the axial force,
**characterized in that**
the controller (120) is configured to cause the rotor (122), when performing the remedial action, to retract proximally a predetermined distance.

2. The pullback and rotation unit (112) of claim 1, wherein the at least one sensor (128) is mounted on the rotor (122).

3. The pullback and rotation unit (112) of claim 2, wherein the rotor (122) includes a main body and a coupling (125) mounted on the main body of the rotor (122), wherein the coupling (125) is configured to couple with the proximal end of the torque cable (104), and wherein the at least one sensor (128) is mounted between the coupling (125) and the main body of the rotor (122).

4. The pullback and rotation unit (112) of claim 3, wherein the coupling (125) is mounted to be movable longitudinally relative to the main body of the rotor (122).

5. The pullback and rotation unit (112) of claim 3, wherein the at least one sensor (128) is mounted on the main body of the rotor (122).

6. The pullback and rotation unit (112) of claim 1, wherein the at least one sensor (128) includes a plurality of sensors (128).

7. The pullback and rotation unit (112) of claim 6, wherein the plurality of sensors (128) are radially spaced from an axis of rotation of the rotor (122).

8. The pullback and rotation unit (112) of claim 1, wherein the predetermined threshold is set below a level at which damage to components of the catheter (100) will occur.

9. The pullback and rotation unit (112) of claim 3, wherein the predetermined threshold corresponds to a force greater than an average axial force applied to the coupling (125) during normal operation of the catheter (100).

10. The pullback and rotation unit (112) of claim 1, wherein the output from the at least one sensor (128) is an average of a plurality of outputs from the at least one sensor (128) over a period of time.

11. The pullback and rotation unit (112) of claim 10, wherein the period of time corresponds to at least one full rotation of an imaging tip (117) located at a distal end of the torque cable (104).

12. The pullback and rotation unit (112) of claim 6, wherein each of the plurality of sensors (128) provides an output indicative of an axial force, and wherein the controller (120) is configured to cause the rotor (122) to perform the remedial action if an average of the outputs from the plurality of sensors (128) exceeds the predetermined threshold.

13. The pullback and rotation unit (112) of claim 1, wherein the predetermined distance is sufficient to relieve at least the portion of the axial force applied to the rotor (122) by the torque cable (104).

14. The pullback and rotation unit (112) of claim 1, wherein the controller (120) is configured to cause the rotor (122), when performing the remedial action, to continue rotating as it is retracted proximally the predetermined distance.

15. An intravascular imaging catheter system (110) comprising:
the pullback and rotation unit (112) of claim 1; and
an imaging catheter (100) including an elongate outer sheath (102) having a proximal end coupled with the housing (115), a torque cable (104) disposed in the outer sheath (102) and having a proximal end coupled with the rotor (122), and an imaging tip (117) located at a distal end of the torque cable (104).

16. The intravascular imaging catheter system (110) of claim 15, wherein the imaging tip (117) includes at least one imaging component selected from the group comprising an optical element (119, 121) and an ultrasonic transducer (127).

## Patentansprüche

1. Rückzugs- und Rotationseinheit (112) zum axialen Verschieben und Drehen eines Drehmomentkabels (104) in einer länglichen Außenhülle (102) eines Katheters (100), wobei die Rückzugs- und Rotationseinheit (112) Folgendes umfasst:
ein Gehäuse (115) mit einer Längsachse;
einen Rotor (122), der in dem Gehäuse (115) angeordnet und montiert ist, um sich relativ zu der Längsachse des Gehäuses (115) zu drehen und linear zu verschieben, wobei der Rotor (122) ferner konfiguriert ist, um mit einem proximalen Ende des Drehmomentkabels (104) gekoppelt zu werden;
mindestens einen Sensor (128), der konfiguriert ist, um eine Ausgabe bereitzustellen, die eine axiale Kraft angibt, die durch das Drehmomentkabel (104) auf den Rotor (122) ausgeübt wird; und
eine Steuerung (120), die konfiguriert ist, um, wenn die Ausgabe einen vorbestimmten Schwellenwert überschreitet, den Rotor (122) zu veranlassen, automatisch eine Abhilfemaßnahme durchzuführen, um mindestens einen Teil der axialen Kraft zu entlasten,
**dadurch gekennzeichnet, dass**
die Steuerung (120) konfiguriert ist, um den Rotor (122) zu veranlassen, sich proximal um eine vorbestimmte Strecke zurückzuziehen, wenn die Abhilfemaßnahme durchgeführt wird.

2. Rückzugs- und Rotationseinheit (112) nach Anspruch 1, wobei der mindestens eine Sensor (128) an dem Rotor (122) montiert ist.

3. Rückzugs- und Rotationseinheit (112) nach Anspruch 2, wobei der Rotor (122) einen Hauptkörper und eine Kopplung (125) beinhaltet, die an dem Hauptkörper des Rotors (122) montiert ist, wobei die Kopplung (125) konfiguriert ist, um mit dem proximalen Ende des Drehmomentkabels (104) gekoppelt zu werden, und wobei der mindestens eine Sensor (128) zwischen der Kopplung (125) und dem Hauptkörper des Rotors (122) montiert ist.

4. Rückzugs- und Rotationseinheit (112) nach Anspruch 3, wobei die Kopplung (125) montiert ist, um in Längsrichtung relativ zu dem Hauptkörper des Rotors (122) beweglich zu sein.

5. Rückzugs- und Rotationseinheit (112) nach Anspruch 3, wobei der mindestens eine Sensor (128) an dem Hauptkörper des Rotors (122) montiert ist.

6. Rückzugs- und Rotationseinheit (112) nach Anspruch 1, wobei der mindestens eine Sensor (128) eine Vielzahl von Sensoren (128) beinhaltet.

7. Rückzugs- und Rotationseinheit (112) nach Anspruch 6, wobei die Vielzahl von Sensoren (128) radial von einer Rotationsachse des Rotors (122) beabstandet ist.

8. Rückzugs- und Rotationseinheit (112) nach Anspruch 1, wobei der vorbestimmte Schwellenwert unter einem Niveau eingestellt ist, bei dem eine Beschädigung von Komponenten des Katheters (100) auftreten wird.

9. Rückzugs- und Rotationseinheit (112) nach Anspruch 3, wobei der vorbestimmte Schwellenwert einer Kraft entspricht, die größer als eine durchschnittliche axiale Kraft ist, die während des normalen Betriebs des Katheters (100) auf die Kupplung (125) ausgeübt wird.

10. Rückzugs- und Rotationseinheit (112) nach Anspruch 1, wobei die Ausgabe von dem mindestens einen Sensor (128) ein Durchschnitt einer Vielzahl von Ausgaben von dem mindestens einen Sensor (128) über einen Zeitraum ist.

11. Rückzugs- und Rotationseinheit (112) nach Anspruch 10, wobei der Zeitraum mindestens einer vollständigen Drehung einer Bildgebungsspitze (117) entspricht, die sich an einem distalen Ende des Drehmomentkabels (104) befindet.

12. Rückzugs- und Rotationseinheit (112) nach Anspruch 6, wobei jeder der Vielzahl von Sensoren (128) eine Ausgabe bereitstellt, die eine axiale Kraft angibt, und wobei die Steuerung (120) konfiguriert ist, um den Rotor (122) zu veranlassen, die Abhilfemaßnahme durchzuführen, wenn ein Durchschnitt der Ausgaben von der Vielzahl von Sensoren (128) den vorbestimmten Schwellenwert überschreitet.

13. Rückzugs- und Rotationseinheit (112) nach Anspruch 1, wobei der vorbestimmte Abstand ausreicht, um mindestens den Teil der axialen Kraft zu entlasten, die durch das Drehmomentkabel (104) auf den Rotor (122) ausgeübt wird.

14. Rückzugs- und Rotationseinheit (112) nach Anspruch 1, wobei die Steuerung (120) konfiguriert ist, um den Rotor (122) zu veranlassen, sich weiter zu drehen, wenn er proximal um die vorbestimmte Strecke zurückgezogen wird, wenn die Abhilfemaßnahme durchgeführt wird.

15. Intravaskuläres Bildgebungskathetersystem (110), das Folgendes umfasst:
die Rückzugs- und Rotationseinheit (112) nach Anspruch 1; und
einen Bildgebungskatheter (100), der eine längliche Außenhülle (102), die ein proximales Ende aufweist, das mit dem Gehäuse (115) gekoppelt ist, ein Drehmomentkabel (104), das in der Außenhülle (102) angeordnet ist und ein proximales Ende aufweist, das mit dem Rotor (122) gekoppelt ist, und eine Bildgebungsspitze (117), die sich an einem distalen Ende des Drehmomentkabels (104) befindet, umfasst.

16. Intravaskuläres Bildgebungskathetersystem (110) nach Anspruch 15, wobei die Bildgebungsspitze (117) mindestens eine Bildgebungskomponente umfasst, die aus der Gruppe ausgewählt ist, die ein optisches Element (119, 121) und einen Ultraschallwandler (127) umfasst.

## Revendications

1. Une unité de retrait et de rotation (112) pour la rotation et la translation axiales d'un câble de torsion (104) dans une gaine externe allongée (102) d'un cathéter (100), l'unité de retrait et de rotation (112) comprenant :
un boîtier (115) avec un axe longitudinal ;
un rotor (122) disposé dans le boîtier (115) et monté pour tourner et se translater linéairement par rapport à l'axe longitudinal du boîtier (115), le rotor (122) étant en outre configuré pour être couplé à une extrémité proximale du câble de torsion (104) ;
au moins un capteur (128) configuré pour produire une sortie représentative d'une force axiale exercée par le câble de torsion (104) sur le rotor (122) ; et
un contrôleur (120) configuré pour, lorsque la sortie dépasse un seuil prédéterminé, faire en sorte que le rotor (122) effectue automatiquement une action correctrice pour atténuer au moins une partie de la force axiale,
**caractérisée en ce que**
le contrôleur (120) est configuré pour faire en sorte que le rotor, lorsqu'il effectue l'action correctrice, se rétracte d'une distance prédéterminée en direction proximale.

2. L'unité de retrait et de rotation (112) de la revendication 1, dans laquelle l'au moins un capteur (128) est monté sur le rotor (122).

3. L'unité de retrait et de rotation (112) de la revendication 2, dans laquelle le rotor (122) comprend un corps principal et un couplage (125) monté sur le corps principal du rotor (122), dans lequel le couplage (125) est configuré pour se coupler avec l'extrémité proximale du câble de torsion (104), et dans lequel l'au moins un capteur (128) est monté entre le couplage (125) et le corps principal du rotor (122).

4. L'unité de retrait et de rotation (112) de la revendication 3, dans laquelle le couplage (125) est monté pour être mobile longitudinalement par rapport au corps principal du rotor (122).

5. L'unité de retrait et de rotation (112) de la revendication 3, dans laquelle l'au moins un capteur (128) est monté sur le corps principal du rotor (122).

6. L'unité de retrait et de rotation (112) de la revendication 1, dans laquelle l'au moins un capteur (128) comprend une pluralité de capteurs (128).

7. L'unité de retrait et de rotation (112) de la revendication 6, dans laquelle la pluralité de capteurs (128) sont espacés radialement par rapport à un axe de rotation du rotor (122).

8. L'unité de retrait et de rotation (112) de la revendication 1, dans laquelle le seuil prédéterminé est paramétré au-dessous d'un seuil auquel apparaîtrait un endommagement de composants du cathéter (100).

9. L'unité de retrait et de rotation (112) de la revendication 3, dans laquelle le seuil prédéterminé correspond à une force supérieure à une force axiale moyenne appliquée au couplage (125) en fonctionnement normal du cathéter (100).

10. L'unité de retrait et de rotation (112) de la revendication 1, dans laquelle la sortie de l'au moins un capteur (128) est une moyenne d'une pluralité de sorties d'au moins un capteur (128) sur une période de temps.

11. L'unité de retrait et de rotation (112) de la revendication 10, dans laquelle la période de temps correspond à au moins une rotation complète d'un embout d'imagerie (117) situé à une extrémité distale du câble de torsion (104).

12. L'unité de retrait et de rotation (112) de la revendication 6, dans laquelle chacun de la pluralité de capteurs (128) produit une sortie représentative d'une force axiale, et dans laquelle le contrôleur (120) est configuré pour faire en sorte que le rotor (122) effectue l'action correctrice si une moyenne des sorties de la pluralité de capteurs (128) dépasse le seuil prédéterminé.

13. L'unité de retrait et de rotation (112) de la revendication 1, dans laquelle la distance prédéterminée est suffisante pour atténuer au moins la partie de la force axiale appliquée au rotor (122) par le câble de torsion (104).

14. L'unité de retrait et de rotation (112) de la revendication 1, dans laquelle le contrôleur (120) est configuré pour faire en sorte que le rotor (122), lorsqu'il effectue l'action correctrice, continue à tourner pendant qu'il est rétracté en direction proximale de la distance prédéterminée.

15. Un système de cathéter d'imagerie intravasculaire (110) comprenant :
l'unité de retrait et de rotation (112) de la revendication 1 ; et
un cathéter d'imagerie (100) comprenant une gaine externe allongée (102) avec une extrémité proximale couplée au boîtier (115), un câble de torsion (104) disposé dans la gaine externe (102) et possédant une extrémité proximale couplée au rotor (122), et un embout d'imagerie (117) situé à une extrémité distale du câble de torsion (104).

16. Le système de cathéter d'imagerie intravasculaire (110) de la revendication 15, dans lequel l'embout d'imagerie (117) comprend au moins un composant d'imagerie sélectionné dans le groupe comprenant un élément optique (119, 121) et un transducteur à ultrasons (127).
